# EUROPEAN PATENT SPECIFICATION

(11) **EP 4 003 585 B1**
(45) Date of publication and mention of the grant of the patent: **29.01.2025**
(21) Application number: 20750183.4
(22) Date of filing: 16.07.2020
(51) Int. Cl.: B01J 19/00, B01J 19/18, C12M 1/00, C12M 1/12, C12N 1/00

(54) **BIOREACTORS FOR GROWING MICRO-ORGANISMS**
BIOREAKTOREN ZUM ZÜCHTEN VON MIKROORGANISMEN
BIORÉACTEURS POUR LA CULTURE DE MICRO-ORGANISMES

(30) Priority: 22.07.2019 FI 20195648
(43) Date of publication of application: 01.06.2022
(73) Proprietor: Solar Foods Oyj, 01730 Vantaa (FI)
(72) Inventor: VAINIKKA, Pasi, 53850 Lappeenranta (FI); PITKÄNEN, Juha-Pekka, 01640 Vantaa (FI)
(74) Representative: Espatent Oy
(86) International application number: PCT/EP2020/070166
(87) International publication number: WO 2021/013698

(56) References cited:
- CN-A- 101 177 660
- CN-U- 201 746 545
- GB-A- 2 305 369
- KR-B1- 100 758 856
- US-A1- 2011 124 091

## Description

### TECHNICAL FIELD

The present disclosure relates generally to growing micro-organisms and more specifically to bioreactors for growing micro-organisms.

### BACKGROUND

Micro-organisms are typically grown in bioreactors under optimally balanced environmental conditions. The optimally balanced environmental conditions include balanced amount of nutrients, gases, heat, pH and pressure. Generally, nutrients and gases are supplied to the bioreactors dissolved in a liquid medium, such as water, broth and the like. However, there are problems associated with bioreactors related to feeding of the gases to the liquid medium and making the gases available for an effective uptake by the micro-organisms.

Conventionally, bioreactors receive the gases from a bottom of the bioreactors, at a defined pressure, into the liquid medium contained in the bioreactor. Typically, the time taken by the gases to rise from the bottom of the bioreactor to a top of the bioreactor (referred to as "residence time") is proportional to the uptake of the gases by the micro-organisms. Furthermore, the gases form bubbles (or gas bubbles) inside the liquid medium, and the size of such gas bubbles has to be minimized in order to maximize an area covered by the gas bubbles so that the gases are taken up by the micro-organisms. Therefore, the bioreactors are provided with various systems, such as gas spargers, pumps, mixers, turbines, different geometrical arrangements (for example U-tube arrangement), and the like, for properly dissolving the gases in the liquid medium, increasing the residence time, and breaking down large sized gas bubbles into small sized gas bubbles (or downsizing). However, despite the various systems for aeration of the liquid medium, a portion of the gases are left undissolved and get accumulated at the top of bioreactors. Moreover, liquid free zones (also referred to as cavitation) are formed in the bioreactor during downsizing of the gas bubbles. As a result, the undissolved gases are unavailable for use by micro-organisms and the liquid free zones negatively affect energy efficiency of the bioreactor and implemented systems thereof.

Further, in order to increase utilization of the gases, a reactor cascade is conventionally used. The reactor cascade employs subsequent bioreactors in series to consume maximum amount of the gases. However, the unused gas in the conventional reactor cascade setup ends in a head-space at the top of each of the individual bioreactors. The unused gases exert pressure on walls of the bioreactors as well as on each other and result in hazards, such as explosion, high reactivity, and the like. Furthermore, such systems can only support growth of slow growing cultures such as mammalian cells but do not support intensive microbial growth.

In a document US3298821A, there is described a process of decomposing waste organic material by subjecting the waste material to aerobic bacterial digestion while moving the waste material horizontally in generally rectangularly cross sectioned beds. The process comprises forming cracks in said beds extending across the entire width of said beds normal to the direction of movement of said beds and extending from the upper surface of said beds downwardly toward the bottom of said beds while preserving the general rectangularly cross sectioned configuration of said beds, whereby efficient and rapid aerobic bacterial digestion may be promoted by subjecting the beds to controlled aeration by passing air through said cracks and around said beds.

In another document CN102191277A, there is described a tray type bioreactor for producing hydrogen. Biomass alternately passes through optical fiber trays and U-shaped heat exchanger trays. Under the bubble effect of reflux hydrogen, a continuous hydrogen producing process is realized by suspension heat exchange and dark-and-light fermentation.

In yet another document WO2014176852A1, there is described a microorganism culture device, comprising a culture box and a bubble generator. The culture box is provided with a bubble guiding structure, wherein bubbles with nutrient content are guided by the bubble guiding structure to move upwards.

In still another document KR101366260B1, there is described a bioreactor apparatus. A reactor provides a purifying treatment space for sewage-waste water. A sewage-waste water inlet pipe and an air inlet pipe are installed at the bottom of the reactor. At least one air diffusing plate is fixed in a cone shape having a slit on the outline and passes the sewage-waste water and air through the slit upward while keeping a part of the air in the lower part. An air outlet pipe and a sewage-waste water outlet pipe are connected to the top end of the reactor.

In yet another document KR20080097893A, there is described a reactor for fermentation of organic waste, the reactor having several chambers. An inlet for refuse and a gas outlet are located at the top of the reactor. An oxygen diffuser and a reaction mixture outlet are located at the bottom of the reactor. An agitating shaft rotates forward and backward in a predetermined angle range. Moreover, a stirrer is provided to branch in left and right directions from a stirring shaft passing through each of media layers formed in the reactor.

In still another document WO2014064338A1, there is described an arrangement for processing organic waste, which comprises a vessel for processing organic waste, an inlet for feeding organic waste into the vessel, an outlet for discharging processed organic waste in the form of reject from the vessel, supporting means comprising at least one support structure for carrying organic waste in said vessel, moving means for moving organic waste on at least one support structure for carrying organic waste in said vessel, supplying means for supplying gas into said vessel, and gas discharging means for discharging exhaust gas. The supplying means are configured to direct gas into organic waste carried on at least one support structure. Said moving means are configured to move organic waste fed via the inlet into the vessel in direction from the inlet to the outlet to at least partly prevent incoming organic waste to mix with organic waste present in the vessel.

In yet another document US3314765A, there is described a bacteriological digester comprising hollow cylinder having an inlet at its top to receive refuse and an outlet at its bottom to discharge the treated organic material. A plurality of horizontal plates within the cylinder subdivide it into a plurality of superimposed drum-shaped compartments. Each plate is formed with at least one transfer port to permit downward flow of organic material. Co-axial rotating shaft means pass through all the compartments from top to bottom of the cylinder.

GB2305369A relates to slurry fermentation processes in which organic or other slurries, such as animal or other wastes are caused, or allowed, to react to produce for example methane and a residue.

Therefore, in light of the foregoing discussion, there exists a need to overcome drawbacks associated with conventional bioreactor designs.

### SUMMARY

The present disclosure seeks to provide a bioreactor for growing micro-organisms in a reaction mixture comprising a reaction medium and micro-organisms. The present disclosure seeks to provide a solution to the existing problem of low dissolution and incomplete utilization of gases in reaction mixture inside a bioreactor. An aim of the present disclosure is to provide a solution that overcomes at least partially the problems encountered in prior art, and provides an efficient and robust design for a bioreactor that achieves higher residence time of the gases and consequently results in a near-complete utilization of the gases in the reaction mixture for optimal growth of micro-organisms.

The present invention provides a bioreactor for growing micro-organisms in a reaction mixture comprising a reaction medium and micro-organisms, the bioreactor comprising
- a first reaction chamber for containing the reaction mixture when in operation, wherein the first reaction chamber has
   - a uniform cross-section and a first volume for containing a first number of micro-organisms,
   - a first input for providing the reaction mixture to the first reaction chamber, and
   - a first output for removing excess gases from the first reaction chamber;
- a second reaction chamber, arranged downstream from the first reaction chamber, for containing the reaction mixture when in operation, wherein the second reaction chamber has
   - a uniform cross-section and a second volume for containing a second number of micro-organisms, the second number of micro-organisms being larger than the first number of micro-organisms,
   - a second input for providing gases to the second reaction chamber, and
   - a second output for removing the reaction mixture from the second reaction chamber; and
- a single means for connecting the first reaction chamber to the second reaction chamber, the means for connecting, when in operation, allows the reaction mixture to flow from the first reaction chamber to the second reaction chamber and the gases to flow from the second reaction chamber to the first reaction chamber;
   wherein
- the means for connecting is the sole input for the gases to the first reaction chamber and the sole input for the reaction mixture to the second reaction chamber, and
- a cross-sectional area of the means for connecting is at most 5 % of a cross-sectional area of one of the first reaction chamber or of the second reaction chamber,
wherein the bioreactor further comprises a collector arranged outside of and on the first reaction chamber and coupled to the first input and the first output, the collector comprises an inlet/outlet to allow the reaction mixture to flow into the first reaction chamber and the excess gases to exit from the first reaction chamber, and the collector is configured to be filled with the reaction mixture to a pre-defined level and with the excess gases on top of the reaction mixture.

Embodiments of the present disclosure substantially eliminate or at least partially address the aforementioned problems in the prior art, and enable effective supply of gases in the bioreactor and enhanced utilization of the gases for optimal growth of micro-organisms. Additionally, the embodiments of the present disclosure enable production of gas bubbles of small size to provide larger surface area for binding of liquid, thereby ensuring proper mixing of the reaction mixture with gases for absorption by the micro-organisms.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1 is a block diagram of a bioreactor, in accordance with an embodiment of the present disclosure;
FIG. 2 is a block diagram of a bioreactor, in accordance with another embodiment of the present disclosure;
FIG. 3 is a schematic illustration of a collector of FIG. 1, in accordance with an embodiment of the present disclosure;
FIG. 4 is a block diagram of a bioreactor, in accordance with yet another embodiment of the present disclosure; and
FIG. 5 is a block diagram of a bioreactor, in accordance with still another embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

The present invention provides a bioreactor for growing micro-organisms in a reaction mixture comprising a reaction medium and micro-organisms, the bioreactor comprising
- a first reaction chamber for containing the reaction mixture when in operation, wherein the first reaction chamber has
   - a uniform cross-section and a first volume for containing a first number of micro-organisms,
   - a first input for providing the reaction mixture to the first reaction chamber, and
   - a first output for removing excess gases from the first reaction chamber;
- a second reaction chamber, arranged downstream from the first reaction chamber, for containing the reaction mixture when in operation, wherein the second reaction chamber has
   - a uniform cross-section and a second volume for containing a second number of micro-organisms, the second number of micro-organisms being larger than the first number of micro-organisms,
   - a second input for providing gases to the second reaction chamber, and
   - a second output for removing the reaction mixture from the second reaction chamber; and
- a single means for connecting the first reaction chamber to the second reaction chamber, the means for connecting, when in operation, allows the reaction mixture to flow from the first reaction chamber to the second reaction chamber and the gases to flow from the second reaction chamber to the first reaction chamber;
wherein
- the means for connecting is the sole input for the gases to the first reaction chamber and the sole input for the reaction mixture to the second reaction chamber, and
- a cross-sectional area of the means for connecting is at most 5 % of a cross-sectional area of one of the first reaction chamber or of the second reaction chamber,
wherein the bioreactor further comprises a collector arranged outside of and on the first reaction chamber and coupled to the first input and the first output, the collector comprises an inlet/outlet to allow the reaction mixture to flow into the first reaction chamber and the excess gases to exit from the first reaction chamber, and the collector is configured to be filled with the reaction mixture to a pre-defined level and with the excess gases on top of the reaction mixture.

The present disclosure provides the aforementioned bioreactor for growing micro-organisms in the reaction mixture comprising the reaction medium and micro-organisms. The bioreactor of the present disclosure comprises two or more reaction chambers arranged vertically (downstream) with respect to each other, connected by the means for connecting. The reaction chambers, arranged in the downstream arrangement, allow reaction medium and gases to flow in a counter-flow direction. The counter-flow direction enables longer residence time of gases in the reaction mixture so that the gases are efficiently consumed for optimal growth of the micro-organisms. The bioreactor is arranged to form gas bubbles of optimal sizes, which further enables in efficient consumption of the gases for optimal growth of the micro-organisms. Beneficially, the bioreactor setup and the counter-flow of the reaction mixture and the gases results in maintaining different stages of growth in different reaction chambers, such that growth starts at a top-most reaction chamber and advanced growth is in a lowest chamber. Moreover, the bioreactor provides efficient withdrawal of micro-organisms upon growth, for providing an overall energy efficient bioreactor for growing micro-organisms. Additionally, a vessel, namely a collector, is arranged with the bioreactor to collect excess gases only at a top of the collector with a nominal volume, which makes the bioreactor safer.

The present disclosure provides the bioreactor for growing micro-organisms in the reaction mixture comprising the reaction medium and micro-organisms. Throughout the present disclosure, the term *"bioreactor"* refers to a vessel intended for biological and/or biochemical reactions required for culturing cells, growing micro-organisms, and production of biomolecules of pharmaceutical interest therefrom, under defined and controlled physical and chemical conditions. The biomolecules of pharmaceutical interest may be selected from a group comprising, but not limited to, vaccines, drugs, hormones, enzymes, antibodies, biopharmaceuticals, plasmid DNA, viruses, phage, proteins, peptides, and lipids, grown using processes adhering to good manufacturing practices under good manufacturing practice (GMP) conditions. The bioreactor may have a shape such as cylindrical, conical, cuboidal or cubical. Optionally, the shape of the bioreactor may be selected based on a desired volume and usage of the bioreactor. For example, a cylindrical bioreactor has more volume as compared to a cuboidal bioreactor of same height and cross-sectional area. Optionally, the volume of the bioreactor is for example 10 litres, 100 litres, 200 litres, 1000 litres, 5000 litres, 10000 litres, 20000 litres, 50000 litres, 100000 litres, 200000 litres and the like.

In an embodiment, the bioreactor is fabricated from a material that is inert to contents to be processed in the bioreactor. In an example, the material used for fabrication may be stainless steel (for example type 304, 316 or 316 litres), other suitable metals or alloys, glass material, fibres, ceramic, plastic materials and/or combinations thereof. Moreover, the fabrication material is typically waterproof and strong enough to withstand abrasive effects of various biological, biochemical and/or mechanical processes, such as micro-organisms concentrations, biomass productions, agitation forces, aeration forces, operating pressures, temperatures, acids, alkali and so forth. Typically, the bioreactor has an adequate thickness to hold a weight of the reaction mixture, and carry out various biological, biochemical and/or mechanical processes. In an example, bioreactors holding a large weight of reaction mixture may thereby have large thickness. Furthermore, the bioreactor should preferably be such that it withholds the sterilisation conditions, for example steam sterilisation with water vapour at 121 °C and a pressure of 2.5 bar.

Throughout the present disclosure, the term *"reaction mixture"* refers to constituents, to be fed inside the reaction chamber, employed for growth of micro-organisms. The reaction mixture comprises a reaction medium and micro-organisms. The reaction medium includes a liquid phase with or without nutrients and serves as a nutrient and growth medium for micro-organisms. Optionally, the liquid phase is selected from a group comprising water, sea water, brackish water, recycled process waters such as dairy run-off, saline media, and/or combinations thereof. Furthermore, the liquid phase may comprise added nutrients, including carbon, magnesium, potassium, phosphorus, sulphur, iron, zinc, manganese, nitrogen (for example in the form of ammonia, urea, nitrate, nitrite, amino acids, proteins (soluble, insoluble or hydrolysed)), animal by-products, dairy wastes, yeast, fatty acids, alcohols, polysaccharides, minerals, vitamins, growth factors, acids, bases, antibiotics, anti-foam agents, surfactants and the like.

It will be appreciated that the reaction mixture comprises an inoculum of micro-organisms that work as starting material for the generation of more micro-organisms under optimal growth conditions. The term *"micro-organisms"* refers to algae, bacteria, cyanobacteria, yeast, fungi, archaea and the like. The bioreactor provides defined and controlled physiological conditions required for the growth of micro-organisms. Optionally, the bioreactor is used to culture eukaryotic cells, including plant cells, fungus, hybridoma cell lines and so forth. Initially, the bioreactor is seeded with a volume of inoculum from an aseptically maintained microbial culture. Further, the micro-organisms are allowed to grow, in a controlled environment, for a defined period of time to achieve an optimum growth. The optimal growth of the micro-organisms pertains to its biomass or byproduct of the microbial growth, to be subsequently harvested for later use. Optionally, the reaction mixture is prepared outside the bioreactor, such as in a sterile shake flask, under aseptic conditions, and then transferred to the reaction chamber under aseptic conditions.

According to an embodiment, the gases include, but are not limited to, oxygen, carbon dioxide, carbon monoxide, nitrogen, hydrogen, inert gases, oxides of nitrogen, methane, and so forth. It will be appreciated that apart from nutrient medium the micro-organisms require gases for optimal growth. The gases are dissolved in the reaction mixture inside the bioreactor. For example, oxygen is provided to the micro-organisms in dissolved form. Typically, the dissolved oxygen is provided to micro-organisms continuously through a process called aeration. Dissolving gases in the reaction mixture is proportional to residence time of gases. Throughout the disclosure, the term *"residence time"* as used herein refers to a duration of time which the gases spend in a bioreactor. In an example, the residence time of a gas, in the form of a gas bubble, in the reaction mixture may range from 10 to 30 minutes. Optionally, small gas bubbles have higher surface area per volume as compared to large gas bubble and therefore have less buoyancy and therefore have longer residence time.

Throughout the present disclosure, the term *"reaction chamber"* refers to a vessel, inside the bioreactor, in which the biological and/or biochemical reactions are carried out. The reaction chamber may have a specific shape such as cylindrical, conical, cuboidal or cubical. Typically, the reaction chamber is a three-dimensional hollow structure or container having a specific volume. For example, the three-dimensional volume may be defined by dimensional parameters, such as length, height, width, diameter and the like. Moreover, the reaction chamber includes a uniform cross-section.

The bioreactor comprises the first reaction chamber and the second reaction chamber. Typically, the bioreactor comprises two (i.e. the first and second) or more reaction chambers. Further, the first and second reaction chambers are structurally similar to each other, for example, based on dimension, material for construction and the like. Alternatively, the first and second reaction chambers may be structurally dissimilar. It will be appreciated that first and the second reaction chambers may be functionally similar to each other. Further, based on the number of reaction chambers in the bioreactor the residence time of gases in the reaction mixture may be increased, which in turn may increase dissolution of the gases in the reaction mixture thereby enabling optimal growth of micro-organisms in the bioreactor. Further, the bioreactor may include smaller (for example, 2 to 10) or larger (for example, 11-20) number of reaction chambers based on the desired production or output of the micro-organisms.

The first reaction chamber contains the reaction mixture, when in operation. It will be appreciated that the term *"when in operation"* used in the present disclosure is to be interpreted to not limit the bioreactor only when it is operated by a given user but is intended to include both a structural as well as functional aspect of the bioreactor. Further, the first reaction chamber during operation includes the micro-organisms in at least one growth phase of a lag phase (where the growth of the micro-organisms is about to start or just started), a log phase (where growth of the micro-organisms is increasing at a fast rate), or a stationary phase (where growth has stopped or is about to stop). Alternatively, in the case of continuous cultivations where growth medium is added constantly and reaction mixture is removed constantly, the growth of the micro-organism can reach a steady-state where the growth rate is constant.

The first reaction chamber has the first volume for containing the first number of micro-organisms. The term *"first volume"* refers to a space inside the first reaction chamber created based on dimensions of the first reaction chamber. In an example, a first reaction chamber having a shape of a cylinder includes a first volume, which is based on a height and a radius of the first reaction chamber. In another example, when a first reaction chamber includes a shape of cuboid, the first volume is based on a length, a dimension and a height of the first reaction chamber. Further, it will be appreciated that during operation the reaction mixture, is provided or fed into the first reaction chamber, having the first number of micro-organisms. Typically, the first number of micro-organisms is an number or percentage of micro-organisms present in the reaction mixture contained in the first reaction chamber.

The first reaction chamber has the first input for providing the reaction mixture to the first reaction chamber. Optionally, the first input is a tubular structure having a certain length, which acts as inlet for providing the growth medium to the first reaction chamber. The growth medium for the first input may be provided from a growth medium storage unit, which may be situated outside the bioreactor. Further, a pump may be employed to allow the flow the growth medium from the reaction growth medium unit to the first reaction chamber. Additionally, the first input may be operatively coupled to a regulator for regulating supply of the growth medium via the first input into the first reaction chamber. The cells are typically fed to the first reaction chamber once at the beginning of the process, via a separate feed inlet.

The first reaction chamber has the first output for removing excess gases from the first reaction chamber. The first output, similar to the first input, is a tubular structure having a certain length, which acts as an outlet for the excess gases from the first reaction chamber. Optionally, the first output may be operatively coupled to a regulator for regulating outflow of the excess gases via the first output. Throughout the present disclosure, the term *"excess gases"* refers to a portion or amount of the gases that are in excess or unused, i.e. not consumed by the micro-organisms. Typically, the gases are provided to the bioreactor based on a predetermined quantity, which is based on a number of micro-organisms present in the reaction mixture. In other words, based on a desired quantity of the micro-organisms to be grown and knowing the ability of the micro-organism to utilise the gases, the quantity of gases and the reaction mixture is decided. Moreover, excess gases are the gases which do not get mixed properly in the reaction mixture in the bioreactor and therefore are not available for consumption by the micro-organisms. Furthermore, excess gases are the gases which have a larger bubble size and thereby are not able to cover maximum area in the reaction mixture inside in the bioreactor. Optionally, the amount of excess gases depends on different lengths of the growth phase of the micro-organisms. Optionally, the amount of excess gases depends on strategies utilized for growing of micro-organisms such as: aerobic growth, anaerobic growth, microaerobic growth and so forth. Further, the excess gases of the second reaction chamber is received by the first reaction chamber. Subsequently, the excess gases removed from the first reaction chamber may be stored in an excess gas storage unit arranged between the first output and the second input.

The bioreactor further comprises a collector arranged outside of and on the first reaction chamber and coupled to the first input and the first output. The term *"collector"* refers to a container or a vessel, having a size substantially smaller as compared to the size of the reaction chamber, and arranged outside of and on the first reaction chamber. The collector is arranged downstream from the first input and the first output, i.e. arranged between the first reaction chamber, and the first input and the first output. The collector comprises an inlet/outlet to allow the reaction mixture to flow into the first reaction chamber and the excess gases to exit from first reaction chamber.

The collector is configured to be filled with the reaction mixture to a pre-defined level and with the excess gases on top of the reaction mixture. Typically, the reaction mixture is fed into the collector by the first inlet, such that the reaction mixture is received by the first reaction chamber and thereafter by the second reaction chamber. Once the first and second reaction chambers are filled, the collector also gets filled. It will be appreciated that the flow of the reaction mixture may be regulated such that the first and second reaction chambers are completely filled, and the collector is partially filled with the reaction mixture. Therefore, the pre-defined level of the reaction mixture in the collector may relate to a state when the reaction mixture occupies one half of the volume of the collector and the excess gases occupy another half of the volume of the collector. It will be appreciated that the excess gases occupy a space above the reaction mixture in the collector. The collector is configured in a manner such that a headspace for the excess gases in the collector is small, which in turn minimizes accumulation of excess gases in the collector. In deed the collector provides two different technical effects. Firstly the collector enables passage of the reaction mixture into the first reaction chamber and passage of the excess gases exiting out of the first reaction chamber. Secondly, the collector functions as a vessel that is configured to be filled with the reaction mixture to a pre-defined level, so that the excess gases are on top of the reaction mixture (namely, in a remaining volume of the collector). In operation, the first reaction chamber and a second reaction chamber are completely filled with the reaction mixture, and the collector is partially filled to the pre-defined level with the reaction mixture, thereby allowing the excess gases to occupy only the remaining volume of the collector. The excess gases are potentially hazardous, namely due to undesirable reactions occurring upon their mixing. Thus, by employing the collector, an accumulation of such potentially hazardous gases in the reaction chambers is regulated effectively. Technical effect of the collector is thus to regulate accumulation of potentially hazardous gases in the first reactor chamber.

The collector comprises an inlet/outlet to allow the reaction mixture to flow into the first reaction chamber and the excess gases to exit from first reaction chamber. More optionally, the collector comprises a coarse filter in the inlet/outlet to enable the collector to remove coarse suspended particles that causes deposits on the reaction chamber causing scaling and/or corrosion of the reaction chambers. More optionally, the coarse filter is mounted on a separate opening, which is arranged at a bottom end of the collector connected to the first reaction chamber.

The second reaction chamber is arranged downstream from the first reaction chamber. The second reaction chamber contains the reaction mixture, when in operation. It will be appreciated that the term *"when in operation"* used in the present disclosure is to be interpreted to not limit the bioreactor only when it is operated by a given user but is intended to include both a structural as well as functional aspect of the bioreactor. The second reaction chamber is vertically positioned lower to the first reaction chamber. The aforesaid arrangement enables the reaction mixture to flow from the first reaction chamber to the second reaction chamber. The said arrangement of the first and second reaction chambers also allows the gases to flow from the second reaction chamber to the first reaction chamber (which will be explained in greater detail, herein later). The reaction mixture flows from the first reaction chamber to the second reaction chamber due to effect of gravity. However, the gases flow from the second reaction chamber to the first reaction chamber due to effect of gravity and buoyancy.

Optionally, the first reaction chamber is positioned on top of the second reaction chamber in a manner such that both the first and second reaction chambers share a common vertical axis. Alternatively, the first reaction chamber may be positioned on top of the second reaction chamber such that they do not share the common vertical axis, i.e. the second reaction chamber is positioned adjacent and downstream from the first reaction chamber. In such a case, the first and the second reaction chambers are vertically spaced apart. In another embodiment, the second reaction chamber is not positioned below the first reaction chamber, rather vertically spaced apart such that a top surface of the second reaction chamber is above a bottom surface of the first reaction chamber.

According to an embodiment, the first reaction chamber is positioned at a first height and the second reaction chamber is positioned at a second height above the base of the bioreactor, preferably the second height is 0, i.e. the second reaction chamber is positioned directly on the base of the bioreactor. Optionally, additional reaction chambers may be arranged between the first reaction chamber and the second reaction chamber in a vertical arrangement with respect to each other and the first and second reaction chambers. In an example, the first reaction chamber is positioned at a height H₁ and the second reaction chamber is positioned at a height H₂ above the base of the bioreactor, such that H₁ is more than H₂. In such an example, a third reaction chamber may be positioned at a height H₃, a fourth reaction chamber is positioned at a height H₄, and a fifth reaction chamber is positioned at a height H₅ above the base of the bioreactor, such that H₃ is less than H₁, H₄ is less than H₃ and H₅ is less than H₄.

The second reaction chamber contains the reaction mixture. As explained herein above, the second reaction chamber receives the reaction mixture from the first reaction chamber. It will be appreciated that in the second reaction chamber the micro-organisms are in a growth phase. Further, the second reaction chamber has the second volume for containing the second number of micro-organisms, the second number of micro-organisms being larger than the first number of micro-organisms at steady-state conditions. The term *"second volume"* refers to a space inside the second reaction chamber created based on dimensions of the second reaction chamber. In an example, the second reaction chamber may include a shape of cylinder having the second volume based on a height and a radius of the second reaction chamber. Further, as mentioned herein above the second reaction chamber may be structurally identical to or different from the first reaction chamber.

The second number of the micro-organisms in the second reaction chamber is larger than the first number of the micro-organisms in the first reaction chamber. Typically, growth of the micro-organisms in the second reaction chamber is more compared to the growth of micro-organisms in the first reaction chamber, which will be explained in greater detail herein later.

The second reaction chamber has the second input for providing gases to the second reaction chamber. Optionally, the second input is a tubular structure having a certain length, which acts as an inlet for providing the gases to the second reaction mixture. The second input may be provided on side walls of the second reaction chamber. Further, the second input may be provided at a bottom end of the second reaction chamber. The gases for the second input may be provided from a gas storage unit, which may be situated outside the bioreactor. Further, a pump may be employed to allow the flow the gases from the gas storage unit to the second reaction chamber. Additionally, the second input may be operatively coupled to a regulator for regulating supply of the gases via the second input into the second reaction chamber. Optionally, the gases may be stored under pressure in the gas storage unit, i.e. in a compressed state. Moreover, a flowrate of the gases may be controlled by a controller arrangement, i.e. the controller arrangement may regulate a quantity and/or a velocity of the gases to be flown from the gas storage unit to the second reaction chamber. In an example, a flowrate of gas is in a range of 0.1 to 2 volume of gas / volume of reaction mixture / minute (vvm).

Optionally, the second input may comprise a nozzle having a number of openings for creating gas bubbles. The nozzle, for example, a sparger, may serve as a protrusion at the end of the second input, and comprises a number of openings, such as small holes, to disperse the gases as gas bubbles into the second reaction chamber. The gases sparged through the number of openings of the nozzle results in a combination of small and large gas bubbles. Moreover, diameter of the openings in the nozzle can be for example 0.5-200 µm (micrometres), preferably 1-30 µm, more preferably 3-10 µm. The diameter of the openings in the nozzle can be for example from 0.5, 0.7, 1, 2, 3, 5, 7, 10, 15, 20, 22, 25, 30, 35, 40, 45, 50, 55, 60, 65, 70, 75, 80, 85, 90, 95, 100, 110, 120, 130, 140 or 150 µm up to 1, 2, 3, 5, 7, 10, 15, 20, 22, 25, 30, 35, 40, 45, 50, 55, 60, 65, 70, 75, 80, 85, 90, 95, 100, 110, 120, 130, 140, 150, 160, 170, 180, 190 or 200 µm. Optionally, shape of gas bubbles is any of tubular, spherical, hemispherical, ellipsoidal, semi-ellipsoidal and/or a combination thereof. The term *"sparge", "sparged"* or *"sparging"* refers to a process of injecting a gas into a liquid such as the reaction mixture by employing the sparger (or a diffuser or a nozzle). Optionally, sparging is used to dissolve a gas into liquid, such as in aeration and carbonation, for further reaction in applications such as fermentation, ozonation, oxidation, hydrogenation and so forth. Alternatively, sparging is used to remove contaminants from the reaction mixture, such as in stripping applications and so forth.

In an embodiment, the second input comprises a plurality of passages, each for carrying an individual gas of the gases to be provided to the second reaction chamber. In an example, the second input includes at least on separation making two or more passages for the gases. Each of the plurality of passages acts as a dedicated input for carrying the individual gas of the gases to be provided to the second reaction chamber. The dedicated inputs prevent mixing of the gases, which may cause undesirable reactions such as exothermic reaction. In an example, the second input comprises a first passage carrying oxygen gas, a second passage carrying carbon dioxide, a third passage carrying hydrogen gas, and so forth. Optionally, the second input may be formed by a group of second inlets arranged at different positions in the second reaction chamber.

The second reaction chamber has the second output for removing the reaction mixture from the second reaction chamber. The second output, similar to the second input, is a tubular structure having a certain length, which acts as an outlet for the reaction mixture comprising a second volume of micro-organisms from the second reaction chamber. Optionally, the second output may be operatively coupled to a regulator for regulating outflow of the reaction mixture via the second output. Optionally, the second output may be provided on side walls of the second reaction chamber. Further, the second output may be provided at a bottom end of the second reaction chamber and opposite to the second input. Optionally, a pump is employed to remove the reaction mixture from the second reaction chamber. As explained herein above, the second number of micro-organisms in the second reaction chamber is larger than the first number of micro-organisms in the first reaction chamber. It will be appreciated that the reaction mixture to be removed from the second reaction chamber has micro-organisms in advanced growth phase, i.e. the log phase. Specifically, the gases provided by the second inlet majorly comes in contact with the reaction mixture of the second reaction chamber, allowing the micro-organisms present in the reaction mixture of the second reaction chamber to substantially consume the gases and causes substantial growth thereof. Therefore, the micro-organisms at a bottom end of the second reaction chamber acquire better or greater growth rate as compared to the micro-organisms at top end of the second reaction chamber. Optionally, the reaction mixture of the second reaction chamber is constantly removed from the reaction chamber to accommodate reaction mixture of the first reaction chamber, that is in the lag phase or early log phase, to achieve optimal growth in the second reaction chamber. Optionally, the reaction mixture removed from the second reaction chamber is stored in an output reaction mixture storage unit.

In an embodiment, each of the first reaction chamber and the second reaction chamber further comprises an agitator arrangement for mixing the reaction mixture and the gases therein. The term *"agitator arrangement"* as used herein refers to a rotating device for mixing the gases and the reaction mixtures. The agitator arrangement stirs the reaction mixture and the gases, which increases the pressure inside the first and second reaction chambers. Further, the agitator arrangement makes the reaction mixture to flow in a defined direction (away from a centre and in a circular manner), to ensure proper mixing of the reaction mixture with gases. In an example, the agitator arrangement is configured and operable to rotate in a clockwise direction, anti-clockwise direction or both. Further, the rotation speed of the agitator arrangement may be in a range for example 100-1000 rpm or 400-600 rpm. For example, the rotation speed can be from 100, 150, 200, 250, 300, 350, 400, 450, 500, 550, 600, 650, 700 or 750 rpm up to 200, 250, 300, 350, 400, 450, 500, 550, 600, 650, 700, 750, 800, 850, 900, 950 or 1000 rpm.

In an embodiment, the agitator arrangement comprises a motor, a shaft connected to the motor and blades arranged on the shaft. The motor, specifically, a shaft of the motor is directly coupled to an end of the shaft of the agitator arrangement with the help of a shaft coupling for allowing transmission of rotation of the shaft of the motor to the shaft of the agitator arrangement. It will be appreciated that the motor is mounted outside on the first and second reaction chamber, and the shaft and blades disposed inside the first and second reaction chamber. Alternatively, the shaft of the motor may be coupled to the end of the shaft of the agitator arrangement using a belt and pulley arrangement. The blades are rigidly coupled on the shaft of the agitator arrangement, and operable to rotate with the rotation of the shaft. Optionally, the blades comprise a group or a set of blades rigidly coupled to shaft in a vertically spaced apart manner. More optionally, each of the group of blades may include two or more blades. As mentioned herein, each of the first and the second reaction chambers comprises a separate agitator arrangement. Alternatively, the first and the second reaction chambers may include a common agitator arrangement, i.e. having a shaft running through both the first and the second reaction chambers. Further, blades are coupled to the shaft and the shaft is coupled to a motor (either directly or using a belt and pulley arrangement).

In an embodiment, the bioreactor further comprises discs arranged, on the shaft, adjacent to the blades. The discs enable creation of sub-chambers inside each of the first and second reaction chambers. For example, a given disc in the first reaction chamber creates a first sub-chamber and a second sub-chamber. In an embodiment, the discs are configured to have a shape conforming to the cross-sectional area of one of the first and the second reaction chambers. Further, the discs comprise a size smaller than the cross-sectional area creating a narrow space between the discs and the first reaction chamber or the second reaction chamber. The narrow space enables the reaction mixture to flow (in a downward direction due to gravity) between the sub-chambers (i.e. from the first sub-chamber to second sub-chamber). Further, the gases flow (in an upward direction due to buoyancy) between the sub-chambers (i.e. from the second sub-chamber to first sub-chamber). The discs allow increasing the residence time of the gases. Moreover, the discs increase the efficiency of mixing of reaction mixture with the gases.

In an embodiment, the bioreactor further comprises a gas recycle arrangement, arranged between the first output and the second input for recycling the excess gases. It will be appreciated that the gas recycle arrangement essentially includes an elongate passage or tubular structure that fluidically couples the first out of the first reaction chamber to the second input of the second reaction chamber. Optionally, the gas recycle arrangement is coupled to the excess gas storage unit and the gas storage unit. The gas recycle arrangement may include valves and regulator for controlling the flow of excess gases. Additionally, the gas recycle arrangement may include a gas separation arrangement, known in the art. The gas separation arrangement may be operable to separate the mixture of gases into individual gases that are subsequently transmitted to the second input of the second reaction chamber. In an example, the gas recycle arrangement removes any impurities that may be present in the excess gases.

The bioreactor comprises means for connecting the first reaction chamber to the second reaction chamber, the means for connecting, when in operation, allows the reaction mixture to flow from the first reaction chamber to the second reaction chamber and the gases to flow from the second reaction chamber to the first reaction chamber. Throughout the present disclosure, the term *"means for connecting"* refers to a passage, arranged between first and second reaction chambers, to enable flow of the reaction mixture and the gases therebetween. Typically, the bioreactor includes a single means for connecting between the reaction chambers. For example, when the bioreactor comprises the first, the second, a third and a fourth reaction chambers, single and separate means for connecting are arranged between the first and second reaction chambers, the second and third reaction chambers, and the third and fourth reaction chambers.

According to an embodiment, the means for connecting enables in providing the counter-flow to the reaction mixture and the gases, which in turn allows longer residence time of the gases in the bioreactor. The counter-flow of reaction mixture and the gases enable the gases to be consumed efficiently by the micro-organisms for optimum growth thereof. This may further enable in reducing production of the excess gases produced in the reaction chambers.

The means for connecting is the sole input for the gases to the first reaction chamber and the sole input for the reaction mixture to the second reaction chamber. Typically, the means for connecting acts as sole input (or provides sole passage) for the gases to enter into the first reaction chamber from the second reaction chamber. As mentioned herein above, that the second input for the gases is arranged on the second reaction chamber, accordingly the gases tend to move from the second reaction chamber to the first reaction chamber due to gravity and buoyancy. The means for connecting further acts as sole input (or provides sole passage) for the reaction mixture to enter into the second reaction chamber from the first reaction chamber due to gravity. As mentioned herein above, the first input for the reaction mixture is arranged on the first reaction chamber, accordingly the reaction mixture tend to move from the first reaction chamber to the second reaction chamber due to gravity. It will be appreciated that the sole input of the gases would increase the residence time for the gases in the reaction chambers.

Furthermore, cross-sectional area of the means for connecting is at most 5 % of the cross-sectional area of one of the first reaction chamber or the second reaction chamber. It will be appreciated, the means for connecting has a specific size that enables a specific amount of reaction mixture and gases to flow there-through. Further, the size of the means for connecting is substantially smaller as compared to the size of the chambers. Technical effect of selecting size of at most 5 % is to limit flow of gas. If the size is larger than 5% then there is too rapid flow of gas from the second chamber to the first chamber. Too rapid flow of gas does not allow sufficient time for the gas to react in the second reaction chamber.

In an embodiment, the means for connecting the first reaction chamber to the second reaction chamber is a hollow structure having a length L₁. In another embodiment, the means for connecting the first reaction chamber to the second reaction chamber is a pipe having a length L₂. The length L₂ can be larger than the length L₁ above. In an embodiment, the means for connecting is either an elongate or a short hollow structure that acts as the passage between the first and second reaction chambers. Further, the means for connecting may be rigidly coupled to the first and second reaction chambers by way of welding or may be detachably coupled with the help of threads, nuts, screws, rivets and the like. It will be appreciated that based on relative position of the reaction chambers the means for connecting may include varied lengths. For example, when the first and second reaction chambers share common vertical axis and the second reaction chamber is positioned immediately below the first reaction chamber, then the means for connecting is a short hollow structure (such as short hollow cylindrical piece, i.e. with a certain diameter, such as about 10 % of the diameter of the first reaction chamber, for example 5, 7, 10, 12 or 20 % of the diameter of the first reaction chamber) having the length L₁. Alternatively, when the first and second reaction chambers do not share common vertical axis and vertically spaced apart, then the means for connecting is an elongated hollow structure, i.e. a pipe (a tube being a synonym) having the length L₂ that is larger than the length L₁. The diameter of such pipe could be for example about 10 % of the diameter of the first reaction chamber, for example 5, 7, 10, 12 or 20 % of the diameter of the first reaction chamber).

According to an embodiment, bioreactor of the present disclosure may include a single reaction chamber for growing micro-organisms in the reaction mixture. In such a case, the gases and the reaction mixture are provided to the single reaction chamber, i.e. elements such as the first input and output, and the second input and output would be arranged on the single reaction chamber. Further, the bioreactor comprises a single agitator arrangement with respect to the single reaction chamber. As mentioned herein above, the agitator arrangement comprises a shaft, a motor and blades. Moreover, the agitator arrangement further comprises one or more discs arranged on the shaft adjacent to the blades. The discs would create sub-reaction chambers inside the single reaction chamber. Further, the discs have a size smaller than cross-sectional area of the single reaction chamber, thereby creating a narrow passage between the sub-reaction chambers. The narrow passage allows the reaction mixture and the gases to flow between the sub-reaction chambers in the counter-flow direction. In other words, the narrow passage acts as the means for connecting, for the sub-reaction chambers, which enable the counter-flow between the gases and the reaction mixture. Hence, the concentration differences are greater between the top and bottom of the reaction chamber than in a conventional reaction chamber, where there are no discs, but there is still more exchange between the sub-reaction chambers than there would be in a reactor cascade. Further, the discs are operable to rotate along with the blades for mixing of the gases with the reaction mixture inside the single reaction chamber.

In another aspect, an embodiment of the present disclosure provides a method for growing micro-organisms, comprising
- providing a reaction mixture comprising a reaction medium and micro-organisms;
- providing gases;
- mixing the gases with the reaction medium by flowing the gases and the reaction medium in a counter-flow direction; and
- withdrawing grown micro-organisms and/or excess gas.

### DETAILED DESCRIPTION OF THE DRAWINGS

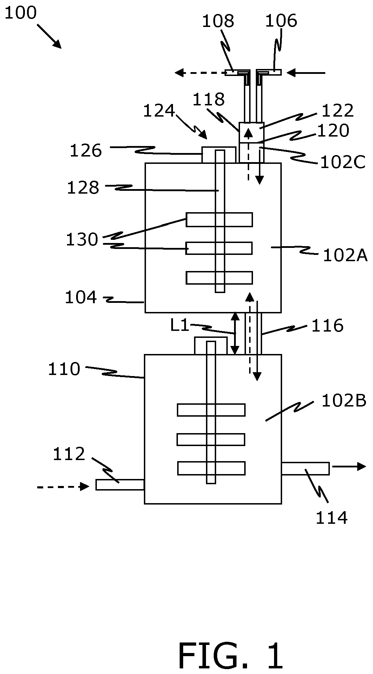

Referring to FIG. 1, there is shown a block diagram of a bioreactor **100** for growing micro-organisms, in accordance with an embodiment of the present disclosure. The bioreactor **100** comprises a first reaction chamber **104** for containing a reaction mixture **102A.** The first reaction chamber **104** has a first volume for containing a first number of micro-organisms, a first input **106** for providing the reaction mixture **102A** to the first reaction chamber **104,** and a first output **108** for removing excess gases (best explained herein later) from the first reaction chamber **104.**

The bioreactor **100** comprises a second reaction chamber **110,** arranged downstream from the first reaction chamber **104,** for containing the reaction mixture **102B.** The second reaction chamber **110** has a uniform cross-section and a second volume for containing a second number of micro-organisms (in the reaction mixture **102B).** The second reaction chamber **110** has a second input **112** for providing gases to the second reaction chamber **110,** and a second output **114** for removing the reaction mixture **102B** from the second reaction chamber **110.**

The bioreactor **100** comprises a single means for connecting **116** the first reaction chamber **104** to the second reaction chamber **110.** The means for connecting **116** allows the reaction mixture **102A** to flow from the first reaction chamber **104** to the second reaction chamber **110** and the gases to flow from the second reaction chamber **110** to the first reaction chamber **104.** The flow of reaction mixture (such as the reaction mixture **102A** and **102B)** is indicated with solid arrow, and the flow of gases is indicated with dashed arrow. As shown, the means for connecting **116** includes a length **L₁**, and the second reaction chamber **110** is positioned immediately below the first reaction chamber **104.**

The bioreactor **100** further comprises a collector **118** arranged outside of and on the first reaction chamber **104** and coupled to the first input **106** and the first output **108** for allowing the reaction mixture (such as the reaction mixture **102)** to flow into the first reaction chamber **104** and the excess gases **122** to exit from first reaction chamber **104,** respectively. As shown, the collector **118** is configured to be filled with a reaction mixture **102C** to a pre-defined level **120** and with the excess gases **122** on top of the reaction mixture **102C.** It will be appreciated that the reaction mixture, i.e. **102A, 102B** and **102C,** is depicted based on its accommodation in the first and second reaction chambers **104, 110,** and the collector **118,** respectively.

Each of the first reaction chamber **104** and the second reaction chamber **110** further comprises an agitator arrangement **124** for mixing the reaction mixture **102A, 102B** and the gases therein. The agitator arrangement **124** comprises a motor **126,** a shaft **128** connected to the motor **126,** and blades **130** arranged on the shaft **128.**

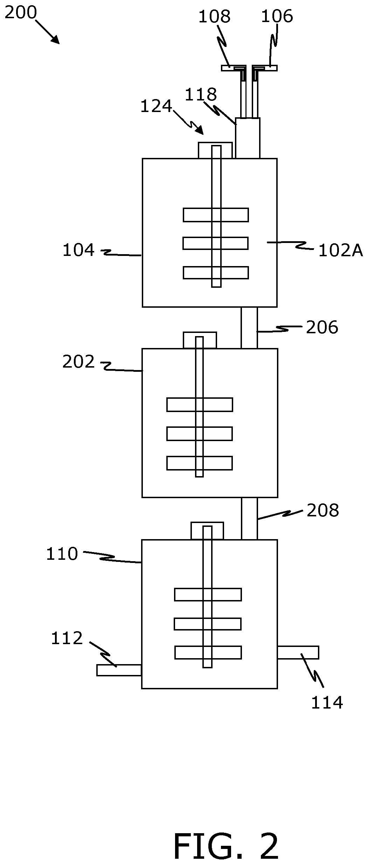

Referring to FIG. 2, there is shown a block diagram of a bioreactor **200** for growing micro-organisms, in accordance with another embodiment of the present disclosure. It will be appreciated that the bioreactor **200** is functionally and substantially structurally similar to the bioreactor **100** of FIG. 1. For example, the bioreactor **200** includes the first reaction chamber **104** having the first input **106** and the first output **108.** The bioreactor **200** also includes the collector **118** arranged outside of and on the first reaction chamber **104** and the agitator arrangement **124.** The bioreactor **200** includes the second reaction chamber **110** having the second input **112** and the second output **114.** However, the bioreactor **200** includes a third (or intermediate) reaction chamber **202** between the first and second reaction chambers **104, 110.** The third reaction chamber **202** includes a third volume for containing a third number of micro-organisms, which is less than the second number of micro-organisms (of the second reaction chamber **110)** and more than first number of micro-organisms (of the first reaction chamber **104).** Further, the first reaction chamber **104** is coupled to the third reaction chamber **202** by means for connecting **206,** and the second reaction chamber **110** is coupled to the third reaction chamber **202** by means for connecting **208.**

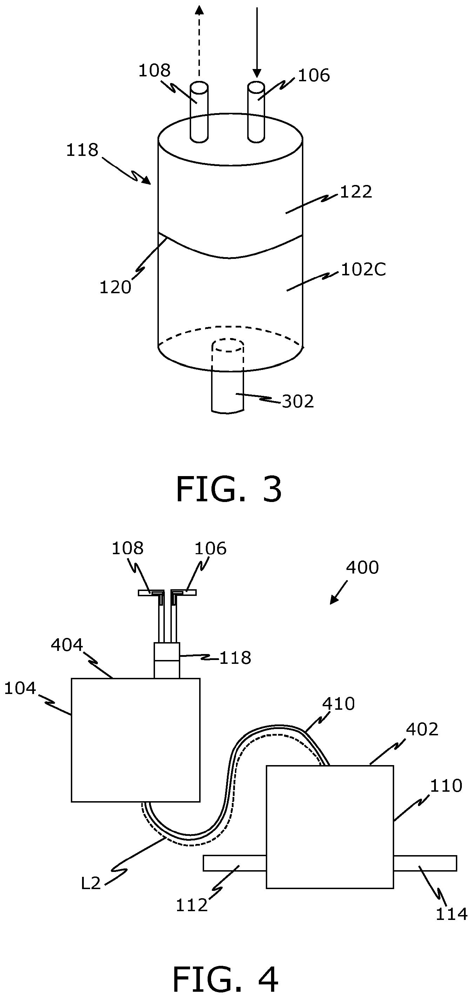

Referring to FIG. 3, there is shown a schematic illustration of the collector **118** of FIG. 1, in accordance with an embodiment of the present disclosure. As shown, the collector **118** is coupled to the first input **106** and the first output **108.** The collector **118,** when in operation, is configured to be filled with the reaction mixture **102C** to a pre-defined level **120,** and with the excess gases **122** on top of the reaction mixture **102C.** The collector **118** comprises an inlet/outlet **302** to allow the reaction mixture **102C** to flow into the first reaction chamber **104** (shown in FIG. 1) and the excess gases to exit from first reaction chamber **104.**

Referring to FIG. 4, there is shown a block diagram a bioreactor **400** for growing micro-organisms, in accordance with yet another embodiment of the present disclosure. It will be appreciated that the bioreactor **400** is functionally and substantially structurally similar to the bioreactor **100** of FIG. 1. For example, the bioreactor **400** includes the first reaction chamber **104** having the first input **106** and the first output **108.** The bioreactor **400** also includes the collector **118** arranged outside of and on the first reaction chamber **104.** Further, the bioreactor **400** includes the second reaction chamber **110** having the second input **112** and the second output **114.** However, the first and the second reaction chambers **104, 110** are vertically spaced apart. Further, the second chamber **110** is not positioned below the first reaction chamber **104** (as shown in FIG. 1) rather vertically spaced apart, and a top surface **402** of the second reaction chamber **110** is above a bottom surface **404** of the first reaction chamber **104.** The bioreactor **400** also includes means for connecting **410,** shown to include a length **L₂**.

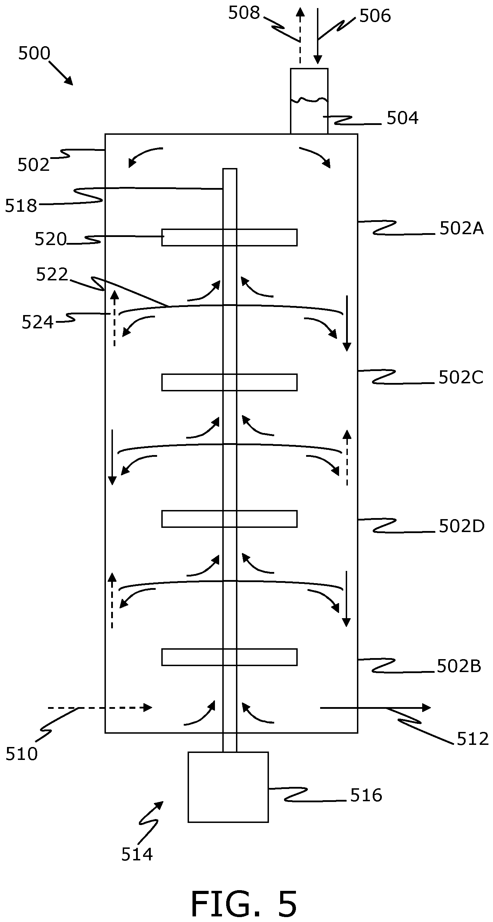

Referring to FIG. 5, there is shown a block diagram a bioreactor **500** for growing micro-organisms, in accordance with still another embodiment of the present disclosure. As shown, the bioreactor **500** includes a single reaction chamber **502** for growing micro-organisms in the reaction mixture **504.** The single reaction chamber **502** comprises a first input **506,** a first output **508,** a second input **510** and a second output **512.** Further, the bioreactor **500** comprises an agitator arrangement **514.** The agitator arrangement **514** comprises a motor **516,** a shaft **518** and blades **520.** The agitator arrangement **514** further comprises one or more discs **522** arranged on the shaft **518** adjacent to the blades **520.** The discs **522** create sub-reaction chambers, depicted as **502A, 502B, 502C** and **502D,** inside the reaction chamber **502.** The discs **522** have a size smaller than cross-sectional area of the reaction chamber **502,** thereby creating a narrow passage **524** between the sub-reaction chambers **502A, 502B, 502C** and **502D.** The narrow passage **524** allows the reaction mixture **504** and the gases to flow between the sub-reaction chambers **502A, 502B, 502C** and **502D** in a counter-flow direction.

Modifications to embodiments of the present invention described in the foregoing are possible without departing from the scope of the present invention as defined by the accompanying claims. Expressions such as "including", "comprising", "incorporating", "have", "is" used to describe and claim the present invention are intended to be construed in a non-exclusive manner, namely allowing for items, components or elements not explicitly described also to be present. Reference to the singular is also to be construed to relate to the plural.

## Claims

1. A bioreactor (100, 200, 400) for growing micro-organisms in a reaction mixture (102A, 102B) comprising a reaction medium and micro-organisms, the bioreactor comprising
- a first reaction chamber (104) for containing the reaction mixture when in operation, wherein the first reaction chamber has
- a uniform cross-section and a first volume for containing a first number of micro-organisms,
- a first input (106) for providing the reaction mixture to the first reaction chamber, and
- a first output (108) for removing excess gases (122) from the first reaction chamber;
- a second reaction chamber (110), arranged downstream from the first reaction chamber, for containing the reaction mixture when in operation, wherein the second reaction chamber has
- a uniform cross-section and a second volume for containing a second number of micro-organisms, the second number of micro-organisms being larger than the first number of micro-organisms,
- a second input (112) for providing gases to the second reaction chamber, and
- a second output (114) for removing the reaction mixture from the second reaction chamber;
- a single means for connecting (116, 206, 208, 410) the first reaction chamber to the second reaction chamber, the means for connecting, when in operation, allowing the reaction mixture to flow from the first reaction chamber to the second reaction chamber and the gases to flow from the second reaction chamber to the first reaction chamber;
wherein
- the means for connecting is the sole input for the gases to the first reaction chamber and the sole input for the reaction mixture to the second reaction chamber,
- a cross-sectional area of the means for connecting is at most 5 % of a cross-sectional area of one of the first reaction chamber or of the second reaction chamber, and
- the bioreactor further comprises a collector (118) arranged outside of and on the first reaction chamber and coupled to the first input and the first output, the collector comprises an inlet/outlet (302) to allow the reaction mixture to flow into the first reaction chamber and the excess gases to exit from the first reaction chamber, and the collector is configured to be filled with the reaction mixture to a pre-defined level (120) and with the excess gases on top of the reaction mixture.

2. A bioreactor (100, 200, 400) according to any of the preceding claims, wherein the second input (112) comprises a plurality of passages, each for carrying an individual gas of the gases to be provided to the second reaction chamber (110).

3. A bioreactor (100, 200, 400) according to any of the preceding claims, further comprising a gas recycle arrangement, arranged between the first output (108) and the second input (112) for recycling the excess gases (122).

4. A bioreactor (100, 200, 400) according to any of the preceding claims, wherein each of the first reaction chamber (104) and the second reaction chamber (110) further comprises an agitator arrangement (124) for mixing the reaction mixture (102A, 102B) and the gases therein.

5. A bioreactor (100, 200, 400) according to claim 4, wherein the agitator arrangement (124) comprises a motor (126), a shaft (128) connected to the motor and blades (130) arranged on the shaft.

6. A bioreactor (100, 200, 400) according to claim 5, further comprising discs arranged on the shaft (128) and adjacent to the blades (130).

7. A bioreactor (100, 200, 400) according to claim 6, wherein the discs are configured to have a shape conforming to the cross-sectional area of one of the first reaction chamber (104) and the second reaction chamber (110), and wherein the discs comprise a size smaller than the cross-sectional area creating a narrow space between the discs and a wall of the first reaction chamber or of the second reaction chamber.

8. A bioreactor (100, 200, 400) according to any of the preceding claims, wherein the means for connecting (116, 206, 208, 410) the first reaction chamber (104) to the second reaction chamber (110) is a hollow structure having a length L1.

9. A bioreactor (100, 200, 400) according to any of the claims 1-7, wherein the means for connecting (116, 206, 208, 410) the first reaction chamber (104) to the second reaction chamber (110) is a pipe having a length L2.

## Patentansprüche

1. Bioreaktor (100, 200, 400) zum Züchten von Mikroorganismen in einer Reaktionsmischung (102A, 102B), die ein Reaktionsmedium und Mikroorganismen umfasst, wobei der Bioreaktor umfasst
- eine erste Reaktionskammer (104) zur Aufnahme des Reaktionsgemischs während des Betriebs, wobei die erste Reaktionskammer aufweist
- einen gleichmäßigen Querschnitt und ein erstes Volumen zur Aufnahme einer ersten Anzahl von Mikroorganismen,
- einen ersten Eingang (106) zur Bereitstellung des Reaktionsgemischs in der ersten Reaktionskammer, und
- einen ersten Ausgang (108) zur Entfernung überschüssiger Gase (122) aus der ersten Reaktionskammer
- eine zweite Reaktionskammer (110), die zur Aufnahme des Reaktionsgemischs während des Betriebs stromabwärts von der ersten Reaktionskammer angeordnet ist, wobei die zweite Reaktionskammer aufweist
- einen gleichmäßigen Querschnitt und ein zweites Volumen zur Aufnahme einer zweiten Anzahl von Mikroorganismen, wobei die zweite Anzahl von Mikroorganismen größer ist als die erste Anzahl von Mikroorganismen,
- einen zweiten Eingang (112) zur Bereitstellung von Gasen in der zweiten Reaktionskammer, und
- einen zweiten Ausgang (114) zum Entfernen des Reaktionsgemischs aus der zweiten Reaktionskammer;
- ein einziges Mittel zum Verbinden (116, 206, 208, 410) der ersten Reaktionskammer mit der zweiten Reaktionskammer, wobei das Mittel zum Verbinden während des Betriebs das Fließen des Reaktionsgemischs von der ersten Reaktionskammer zu der zweiten Reaktionskammer und das Fließen der Gase von der zweiten Reaktionskammer zu der ersten Reaktionskammer ermöglicht;
wobei
- das Mittel zum Verbinden der einzige Eingang für die Gase in der ersten Reaktionskammer und der einzige Eingang für das Reaktionsgemisch in der zweiten Reaktionskammer ist,
- eine Querschnittsfläche der Mittel zum Verbinden höchstens 5 % einer Querschnittsfläche von entweder der ersten Reaktionskammer oder der zweiten Reaktionskammer ist, und
- der Bioreaktor ferner einen Sammler (118) umfasst, der außerhalb und auf der ersten Reaktionskammer angeordnet ist und mit dem ersten Eingang und dem ersten Ausgang gekoppelt ist, wobei der Sammler einen Einlass / Auslass (302) umfasst, um zu ermöglichen, dass das Reaktionsgemisch in die erste Reaktionskammer strömt und die überschüssigen Gase aus der ersten Reaktionskammer austreten, und der Sammler so konfiguriert ist, dass er mit dem Reaktionsgemisch bis zu einem vordefinierten Level (120) und mit den überschüssigen Gasen auf dem Reaktionsgemisch gefüllt zu werden.

2. Bioreaktor (100, 200, 400) nach einem der vorstehenden Ansprüche, wobei der zweite Eingang (112) eine Vielzahl von Durchgängen umfasst, die jeweils dazu da sind, ein einzelnes Gas der Gase, die der zweiten Reaktionskammer (110) zugeführt werden sollen, zu fördern.

3. Bioreaktor (100, 200, 400) nach einem der vorstehenden Ansprüche, ferner umfassend eine Gasrückführungsanordnung, die zwischen dem ersten Ausgang (108) und dem zweiten Eingang (112) angeordnet ist, um die überschüssigen Gase (122) zurückzuführen.

4. Bioreaktor (100, 200, 400) nach einem der vorstehenden Ansprüche, wobei sowohl die erste Reaktionskammer (104) als auch die zweite Reaktionskammer (110) ferner eine Rühranordnung (124) zum Mischen des Reaktionsgemischs (102A, 102B) und der Gase darin umfasst.

5. Bioreaktor (100, 200, 400) nach Anspruch 4, wobei die Rühranordnung (124) einen Motor (126), eine mit dem Motor verbundene Welle (128) und auf der Welle angeordnete Blätter (130) umfasst.

6. Bioreaktor (100, 200, 400) nach Anspruch 5, ferner umfassend Scheiben, die auf der Welle (128) und angrenzend an die Blätter (130) angeordnet sind.

7. Bioreaktor (100, 200, 400) nach Anspruch 6, wobei die Scheiben so konfiguriert sind, dass sie eine Form aufweisen, die der Querschnittsfläche von einer von der ersten Reaktionskammer (104) und der zweiten Reaktionskammer (110) entspricht, und wobei die Scheiben eine Größe aufweisen, die kleiner ist als die Querschnittsfläche, erzeugend einen schmalen Raum zwischen den Scheiben und einer Wand der ersten Reaktionskammer oder der zweiten Reaktionskammer.

8. Bioreaktor (100, 200, 400) nach einem der vorhergehenden Ansprüche, wobei das Mittel zum Verbinden (116, 206, 208, 410) der ersten Reaktionskammer (104) mit der zweiten Reaktionskammer (110) eine Hohlstruktur mit einer Länge L1 ist.

9. Bioreaktor (100, 200, 400) nach einem der Ansprüche 1-7, wobei das Mittel zum Verbinden (116, 206, 208, 410) der ersten Reaktionskammer (104) mit der zweiten Reaktionskammer (110) ein Rohr mit einer Länge L2 ist.

## Revendications

1. Un bioréacteur (100, 200, 400) pour la croissance de micro-organismes dans un mélange réactionnel (102A, 102B) comprenant un milieu réactionnel et des micro-organismes, le bioréacteur comprenant
- une première chambre de réaction (104) destinée à contenir le mélange réactionnel en fonctionnement, la première chambre de réaction ayant
- une section transversale uniforme et un premier volume destiné à contenir un premier nombre de micro-organismes,
- une première entrée (106), pour fournir le mélange réactionnel à la première chambre de réaction, et
- une première sortie (108), pour éliminer les gaz en excès (122) de la première chambre de réaction ;
- une deuxième chambre de réaction (110), agencée en aval de la première chambre de réaction, destinée à contenir le mélange réactionnel en fonctionnement, la deuxième chambre de réaction présentant
- une deuxième section transversale uniforme et un deuxième volume destiné à contenir un deuxième nombre de micro-organismes, le deuxième nombre de micro-organismes étant supérieur au premier nombre de micro-organismes,
- une deuxième entrée (112) destinée à alimenter en gaz la deuxième chambre de réaction, et
- une deuxième sortie (114) destinée à évacuer le mélange réactionnel de la deuxième chambre de réaction ;
- un moyen unique (116, 206, 208, 410) de connexion de la première chambre de réaction à la deuxième chambre de réaction, le moyen de connexion, en fonctionnement, permettant au mélange réactionnel de s'écouler de la première chambre de réaction vers la deuxième chambre de réaction et aux gaz de s'écouler de la deuxième chambre de réaction vers la première chambre de réaction ;
dans lequel
- le moyen de connexion est l'unique entrée pour les gaz dans la première chambre de réaction et l'unique entrée pour le mélange réactionnel dans la deuxième chambre de réaction,
- une surface de section transversale du moyen de connexion est au plus égale à 5 % d'une surface de section transversale de l'une parmi la première chambre de réaction et la deuxième chambre de réaction, et
- le bioréacteur comprend en outre un collecteur (118) agencé à l'extérieur et sur la première chambre de réaction et relié à la première entrée et à la première sortie, le collecteur comprend une entrée/sortie (302) pour permettre au mélange réactionnel de s'écouler dans la première chambre de réaction et aux gaz en excès de sortir de la première chambre de réaction, et le collecteur est configuré pour être rempli du mélange réactionnel jusqu'à un niveau prédéfini (120) et avec les gaz en excès au-dessus du mélange réactionnel.

2. Un bioréacteur (100, 200, 400) selon l'une quelconque des revendications précédentes, dans lequel la deuxième entrée (112) comprend une pluralité de passages, chacun destiné à transporter un gaz individuel parmi les gaz à fournir à la deuxième chambre de réaction (110).

3. Un bioréacteur (100, 200, 400) selon l'une quelconque des revendications précédentes, comprenant en outre un dispositif de recyclage de gaz, agencé entre la première sortie (108) et la deuxième entrée (112) pour recycler les gaz en excès (122).

4. Un bioréacteur (100, 200, 400) selon l'une quelconque des revendications précédentes, dans lequel chacune parmi la première chambre de réaction (104) et la deuxième chambre de réaction (110) comprend en outre un dispositif d'agitation (124) pour mélanger le mélange réactionnel (102A, 102B) et les gaz qu'il contient.

5. Un bioréacteur (100, 200, 400) selon la revendication 4, dans lequel l'agencement d'agitateur (124) comprend un moteur (126), un arbre (128) relié au moteur et des lames (130) agencées sur l'arbre.

6. Un bioréacteur (100, 200, 400) selon la revendication 5, comprenant en outre des disques agencés sur l'arbre (128) et adjacents aux lames (130).

7. Un bioréacteur (100, 200, 400) selon la revendication 6, dans lequel les disques sont configurés pour avoir une forme conforme à la surface de la section transversale de l'une parmi la première chambre de réaction (104) et la deuxième chambre de réaction (110), et dans lequel les disques comprennent une taille inférieure à la surface de la section transversale créant un espace étroit entre les disques et une paroi de la première chambre de réaction ou de la deuxième chambre de réaction.

8. Un bioréacteur (100, 200, 400) selon l'une quelconque des revendications précédentes, dans lequel le moyen de connexion (116, 206, 208, 410) de la première chambre de réaction (104) à la deuxième chambre de réaction (110) est une structure creuse ayant une longueur L1.

9. Un bioréacteur (100, 200, 400) selon l'une quelconque des revendications 1 à 7, dans lequel le moyen de connexion (116, 206, 208, 410) de la première chambre de réaction (104) à la deuxième chambre de réaction (110) est un tuyau ayant une longueur L2.
